# EUROPÄISCHE PATENTSCHRIFT

(11) **EP 3 551 042 B1**
(45) Veröffentlichungstag und Bekanntmachung des Hinweises auf die Patenterteilung: **12.03.2025**
(21) Anmeldenummer: 17818029.5
(22) Anmeldetag: 04.12.2017
(51) Int. Cl.: A61B 5/00, A61B 5/0205, A61B 5/16, A61B 5/01

(54) **EINRICHTUNG ZUR UNTERSUCHUNG EINES MENSCHLICHEN PATIENTEN**
DEVICE FOR EXAMINING A HUMAN PATIENT
DISPOSITIF SERVANT À L'EXAMEN D'UN PATIENT HUMAIN

(30) Priorität: 06.12.2016 AT 511082016
(43) Veröffentlichungstag der Anmeldung: 16.10.2019
(73) Patentinhaber: Schletterer, Heinz, 6235 Reith im Alpbachtal (AT)
(72) Erfinder: Schletterer, Heinz, 6235 Reith im Alpbachtal (AT)
(74) Vertreter: Torggler & Hofmann Patentanwälte - Innsbruck
(86) Internationale Anmeldenummer: PCT/AT2017/060321
(87) Internationale Veröffentlichungsnummer: WO 2018/102841

(56) Entgegenhaltungen:
- WO-A1-2016/074036
- US-A- 5 441 047
- US-A- 5 640 953
- US-A1- 2002 123 704
- US-A1- 2003 216 665
- US-A1- 2004 178 312
- US-A1- 2004 254 501
- US-A9- 2007 058 035

## Beschreibung

Die Erfindung betrifft eine Einrichtung zur Untersuchung eines menschlichen Patienten mit einem Träger für den Patienten, mindestens einer - insbesondere als Bildschirm ausgebildeten - Anzeigevorrichtung, die von dem auf dem Träger befindlichen Patienten einsehbar ist, mindestens zwei Messvorrichtung zum Erfassen von Messdaten des Patienten, die Messdaten bezüglich des Zustands des Patienten ausgeben, sowie einer Auswerteinrichtung, die mit der Anzeigevorrichtung und der Messvorrichtung in Verbindung steht, und die dazu ausgelegt ist, in Abhängigkeit von aus zumindest zwei unterschiedlichen Messvorrichtungen stammenden Messdaten und deren Wechselbeziehung Ausgangssignale zu ermitteln, wobei zumindest eine Anzeigevorrichtung und zumindest ein Sensor einer Messvorrichtung gegenüber dem Träger für den Patienten beweglich gelagert ist.

Einrichtungen zur Untersuchung eines menschlichen Patienten sind bisher hauptsächlich so gehandhabt worden, dass für einzelne körperliche Untersuchungen verschiedene Messungen zeitlich hintereinander vorgenommen wurden und dann die Befunde zu einer Diagnose zusammengetragen worden sind. Die gleichzeitige Berücksichtigung des psychischen Zustandes des Patienten durch Zusammenschau der körperlichen und psychischen Befunde kommt immer mehr Bedeutung zu.

Dahingehend offenbart die US 2002/0123704 A1 einen Massagesessel mit einer Fernbedienung, welche ein Display und mehrere Sensoren aufweist. Wenn eine Person die Fernbedienung in der Hand hält, kommen die Sensoren mit der Haut der Person in Kontakt und können dann entsprechende Messungen durchführen, um den Effekt einer Massage auf die Person zu ermitteln.

Aus der US 4,441,047 A ist ein Gesundheitsüberwachungssystem für Patienten bekannt, mittels welchem der Gesundheitszustand eines Patienten in Ferndiagnose überwacht werden kann.

Solche Vorrichtungen ermöglichen es zwar, verschiedene Messdaten eines Patienten zu ermitteln, eine umfassende Untersuchung des Gesundheitszustands eines Patienten ist aber nicht möglich.

Aufgabe der Erfindung ist es, eine Einrichtung zur Untersuchung eines menschlichen Patienten zu schaffen, mit dem es in kurzer Zeit möglich ist, ein gutes Gesamtbild vom Gesundheitszustand des Patienten zu erlangen.

Erfindungsgemäß wird dies durch die Merkmale des Anspruchs 1 erreicht.

Demgemäß ist vorgesehen, dass die zumindest eine Anzeigevorrichtung und der zumindest eine Sensor einer Messvorrichtung motorisch bewegbar an einer Tragvorrichtung gelagert ist, und dass die zumindest eine Messvorrichtung einen medizinischen Thermoscanner umfasst.

Da die Anzeigevorrichtung und der Sensor der Messvorrichtung motorisch bewegbar an einer Tragvorrichtung gelagert sind, ist ein vollautomatisierter Betrieb mit gleichbleibenden Ergebnissen möglich.

Mit einem Thermoscanner lassen sich verschiedenste körperliche Diagnosen durchführen, insbesondere können Entzündungsherde, aber auch andere Veränderungen aufgezeigt werden.

Die beweglich gelagerte Anzeigevorrichtung kann in mehrfacher Hinsicht verwendet werden. Bei einer rein körperlichen Untersuchung eines Patienten kann die Anzeigevorrichtung in eine für den Patienten bequem einsehbare Lage gebracht werden, sodass er die Untersuchung bzw. die späteren Ergebnisse gut einsehen kann. Dabei kann die Untersuchung selbst durch zumindest einen Sensor einer Messeinrichtung erfolgen, der gegenüber dem auf dem Träger befindlichen Patienten bewegbar gelagert ist.

Weitere vorteilhafte Ausführungsformen sind in den abhängigen Ansprüchen definiert.

Besonders günstig ist eine Ausführungsform, bei der die Anzeigevorrichtung und der Sensor der Messvorrichtung auf einem gemeinsamen Schlitten bewegbar gelagert sind.

Damit kann der Sensor in einer Betriebsstellung körperliche Messdaten aufnehmen bzw. scannen. In einer anderen Betriebsstellung kann der Patient auf den Bildschirm, der in eine geeignete Betriebsstellung gebracht ist, einsehen.

Die Daten am Bildschirm können nicht nur - wie oben ausgeführt - Zustandsdaten bezüglich der durchgeführten Untersuchung oder des Ergebnisses der Untersuchung sein. Vielmehr ist es auch möglich, dem Patienten über die Anzeigevorrichtung bildliche Informationen gegebenenfalls zusammen mit Audioinformationen zuzuspielen und die Reaktion des Patienten dann messtechnisch gleichzeitig oder in einem kurz darauffolgenden Zeitabschnitt zu erfassen.

Damit lassen sich die für die Beurteilung des Gesamtbildes eines menschlichen Patienten immer wesentlicheren mentalen Messdaten neben körperlichen Messdaten eruieren.

Es ist eine Einrichtung zur Untersuchung eines menschlichen Patienten vorgesehen, mit zumindest zwei, vorzugsweise mehreren unterschiedlichen Messvorrichtungen, die Messdaten bezüglich des Zustandes des Patienten ausgeben, wobei eine elektronische Auswerteinrichtung vorgesehen ist, die in Abhängigkeit von aus zumindest zwei unterschiedlichen Messvorrichtungen stammenden Messdaten und deren Wechselbeziehung Ausgangssignale ermittelt.

Die wesentliche Eigenschaft dabei besteht darin, eine Wechselbeziehung zwischen den Messdaten neben den eigentlichen Messdaten selbst auszuwerten. Erst durch diese Korrelation der Messdaten, ist es häufig möglich, zu einer Beurteilung des gesundheitlichen Gesamtbildes zu kommen. Vor allem, wenn ein Teil der korrelierten Messdaten körperliche Messdaten sind, während der andere Teil mentale Messdaten sind. Damit können körperliche und psychische Zustände des Patienten korreliert werden und in Abhängigkeit von dieser Korrelation eine Befundung und/oder eine automatisierte Steuerung von Therapiegeräten erfolgen.

Damit ist die Diagnostik und Therapie in kurzen Zeiträumen wesentlich erleichtert.

Weitere Vorteile und Einzelheiten der Erfindung werden anhand der nachfolgenden Figurenbeschreibung näher erläutert.

Die Figur 1 zeigt ein Ausführungsbeispiel einer erfindungsgemäßen Einrichtung in einer schematischen Darstellung.

Die Figur 2 zeigt verschiedene "biologische "Screening-Positionen" zum Erfassen von körperlichen Messdaten des auf einem Träger liegenden Patienten.

Die Figur 3 zeigt "psychologische, neurologisch und mentale Screening-Positionen" zum Erfassen von psychischen (mentalen) Messdaten des auf dem Träger sitzenden oder liegenden Patienten.

Die Figur 4 zeigt ein Ausführungsbeispiel einer erfindungsgemäßen Einrichtung in einem schematischen Block-Schalt-Diagramm.

Die Figur 5 zeigt in einer schematischen Übersicht die "biologischen Scans" zum Ermitteln von körperlichen Messdaten und "mentale Scans" zum Ermitteln von mentalen Messdaten, die den psychischen Zustand des Patienten wiedergeben.

Die Figuren 6 und 7 zeigen in gesonderten Darstellungen das biologische Screening zum Ermitteln von körperlichen Messdaten und das psychologische Screening zum Ermitteln von mentalen Messdaten, die den psychischen Zustand des Patienten wiedergeben.

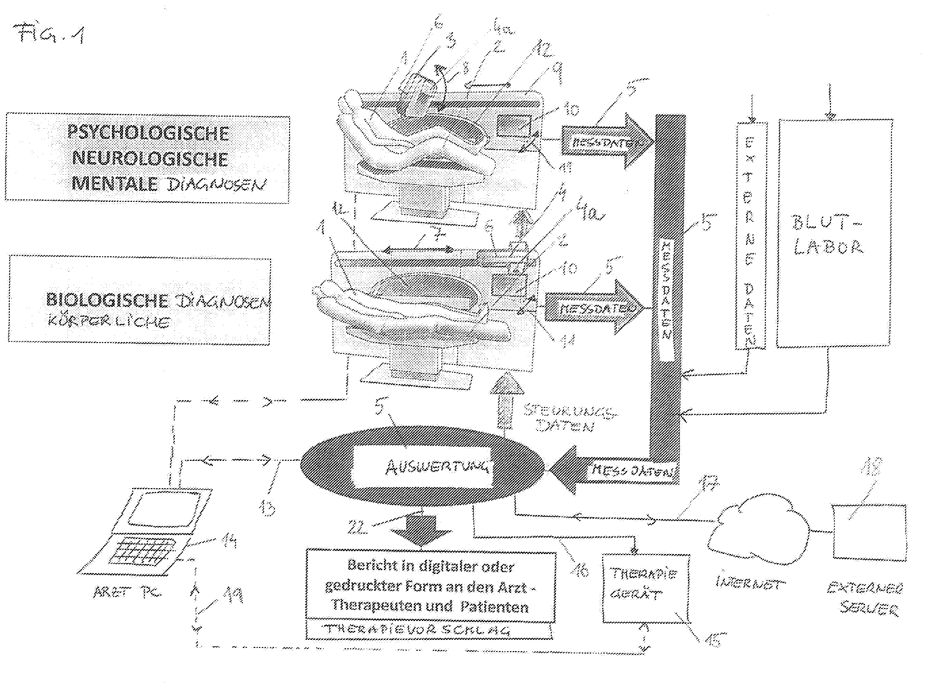

Die Figur 1 zeigt ein Ausführungsbeispiel einer erfindungsgemäßen Einrichtung zur Untersuchung eines menschlichen Patienten 1, der auf einem Träger 2 Platz nehmen kann.

Die beiden übereinander liegenden Darstellungen der verschiedenen Positionen des Trägers 2 betreffen ein- und dieselbe Einrichtung zu zwei verschiedenen Zeiten. In der oberen Position werden psychologische, neurologische, mentale Diagnosen erstellt, in der unteren Position biologische, körperliche Diagnosen.

Es ist eine als Bildschirm ausgebildete Anzeigevorrichtung 3 vorgesehen, die von dem auf dem Träger 2 befindlichen Patienten einsehbar ist (obere Stellung in Figur 1). Weiters ist eine Messvorrichtung 4 zum Erfassen von Messdaten des Patienten 1 sowie eine Auswerteinrichtung 5, die mit der Anzeigevorrichtung 3 und der Messvorrichtung 4 über schematisch dargestellte Leitungen 5 in Verbindung steht.

Beim vorliegenden Ausführungsbeispiel ist sowohl die Anzeigevorrichtung 3 als auch zumindest ein Sensor 4a der Messvorrichtung 4 gegenüber dem Träger 2 für den Patienten 1 beweglich gelagert.

Beim dargestellten Ausführungsbeispiel ist die Messvorrichtung ein Thermoscanner, wobei der Sensor 4a ein Infrarotsensor ist.

Mit einem solchen Thermoscanner lassen sich verschiedenste körperliche Diagnosen durchführen, insbesondere können Entzündungsherde, aber auch andere Veränderungen aufgezeigt werden.

Um den ganzen Körper zu erfassen, ist die Messvorrichtung in Form des Thermoscanners auf einem Halter 6 in Form eines Schlittens montiert und in Richtung der Pfeile 7 längsbeweglich linear geführt.

Der Halter 6 ist ein gemeinsamer Halter für den Thermoscanner 4 und die Anzeigevorrichtung 3. Um den Halter aus der Scanposition in der Mitte der Figur 1 in die obere Position in Figur 1 zu bringen, kann dieser in Richtung der Pfeile 8 verschwenkt werden, womit der Patient Einblick auf die Anzeigevorrichtung bzw. den Bildschirm erlangt. Konstruktiv einfach lässt sich diese in Figur 1 gezeigte Lösung dadurch realisieren, dass der Halter 6 einen verfahrbaren Basisteil und einen an diesem schwenkbar gelagerten Schwenkteil aufweist, der die Anzeigevorrichtung 3 und die Messvorrichtung 4 bzw. deren Sensor 4a trägt.

Der Anzeigevorrichtung kann auch noch mindestens ein (nicht dargestellter) Lautsprecher zugeordnet sein. Dieser kann entweder am feststehenden Teil der Einrichtung oder am verfahrbaren Schlitten angeordnet sein. Es ist auch möglich, dass der Lautsprecher in einem Kopfhörer untergebracht ist.

Insgesamt kann in der in Figur 1 oben gezeigten Position dem Patienten ein audiovisuelles Bild oder eine audiovisuelle Bilderfolge präsentiert werden und anschließend bzw. auch gleichzeitig Messdaten des Patienten erfasst werden. Es handelt sich dabei um reaktive Messdaten, die Aufschluss über den psychologischen Gesundheitszustand des Patienten geben, beispielsweise kann man den Patienten Stresssituationen und Konfliktsituationen vorspielen und seine Reaktion überprüfen. Man kann auch Farben vorspielen und seine Reaktion überprüfen.

Es ist auch möglich, psychologische digitale Fragebögen zu präsentieren, worauf der Patient dann über eine Eingabevorrichtung die Antworten eingeben kann.

Besonders bevorzugt ist eine Ausführungsform, bei der der Bildschirm 3 als Touch-Screen ausgebildet ist und somit gleichzeitig als Eingabevorrichtung dienen kann. Während der psychologischen Untersuchung oder knapp danach, kann beispielsweise über eine zum Eyetracking geeignete Kamera und/oder ein Mikrophon zur Aufnahme von Lauten des Patienten weitere mentale Messdaten aufgezeichnet werden.

Diese Messdaten können bei hintereinanderliegenden Untersuchungen auch zwischengespeichert werden, um die weiter unten beschriebene Korrelation mit anderen Daten durchführen zu können.

Um einen vollautomatisierten Betrieb mit gleichbleibenden Ergebnissen zu erzielen, ist vorgesehen, dass die Anzeigevorrichtung 3 und der Sensor 4a der Messvorrichtung 4 motorisch bewegbar an einer Tragvorrichtung 9 gelagert sind, die in einem optisch ansprechenden Gehäuse untergebracht sein kann.

Die Tragvorrichtung 9 kann auch den Träger 2 für den Patienten tragen. Es ist aber auch möglich, dass dieser Träger 2 wie in Figur 1 dargestellt, direkt auf dem Boden aufsteht. Dann ist die Tragvorrichtung bzw. das Gehäuse 9 und der Träger 2 für den Patienten über den Boden des Raumes miteinander in fixer räumlicher Relation zueinander angeordnet.

Um den Patienten auch während biologischer körperlicher Diagnosen, bei denen der Schlitten als Thermoscanner arbeitet, einen Einblick auf seinen Gesundheitszustand und den Fortschritt der Untersuchung zu ermöglichen, eine weitere Anzeigevorrichtung 10 vorgesehen sein, die in Pfeilrichtung 11 aus dem Gehäuse herausschwenkbar ist bzw. in unbenutztem Zustand wieder bündig mit diesem einschwenkbar ist.

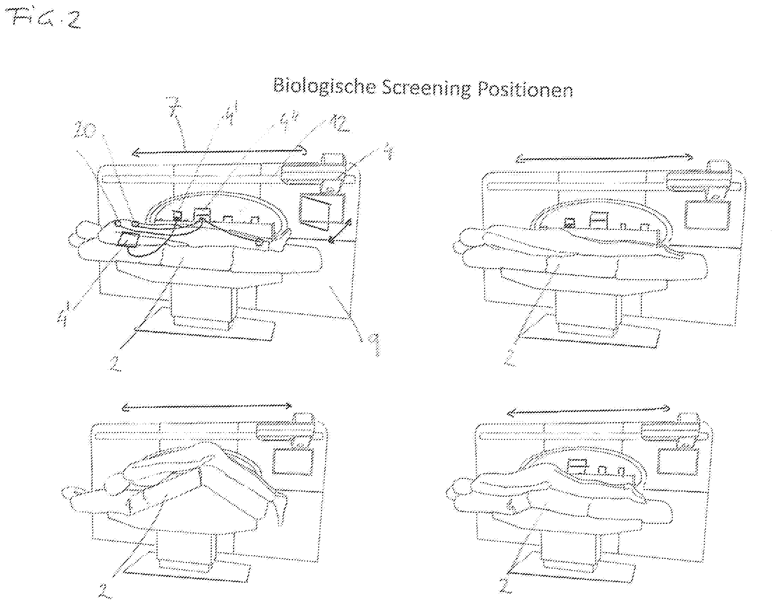

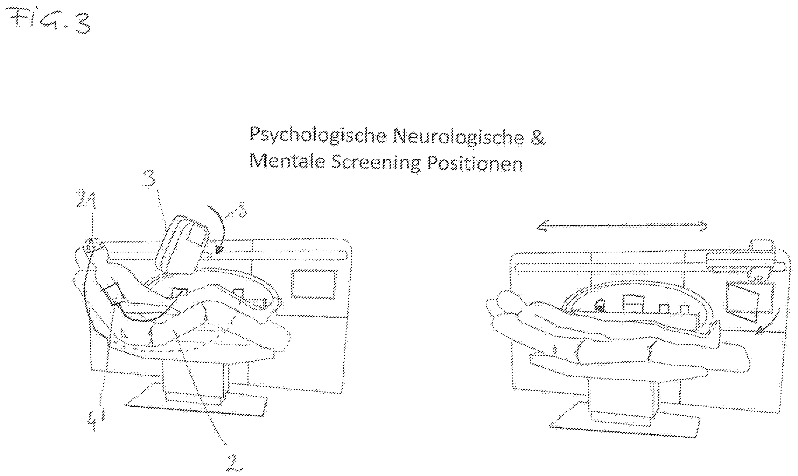

Beim dargestellten Ausführungsbeispiel gibt es dann noch in der Tragvorrichtung 9 bzw. im Gehäuse derselben eine Aufnahme 12, die von der Patientenseite her gut zugänglich ist. In dieser Aufnahme 12 können weitere Sensoren anderer medizinischer Messvorrichtungen untergebracht werden, wie das anhand der folgenden Figuren 2 und 3 noch näher beschrieben werden wird.

Beim dargestellten Ausführungsbeispiel ist der Träger 2 für den Patienten 1 als mehrteilige Sitz-Liege ausgebildet, deren Teile in ihrer Lage motorisch verstellbar sind.

Die Sitzliege ist bevorzugt dreiteilig ausgebildet mit einem Brustteil, einem Mittelteil und einem Fußteil, wie das die Figuren 1 bis 3 klar zeigen.

Die Figur 1 zeigt auch, dass die Sitzliege in eine Sitzstellung (obere Stellung in Figur 1) bringbar ist, wobei die Anzeigevorrichtung heruntergeschwenkt ist, sodass der Kopf des Patienten im Wesentlichen auf derselben Höhe liegt, die die Anzeigevorrichtung. Das entspricht im Wesentlichen der normalen Fernseh- bzw. Bildschirmbetrachtposition. Eine Alternative wäre jene, bei der die Sitzliege und die Anzeigevorrichtung in eine Stellung bringbar sind, in der der Patient im Wesentlichen geradeaus auf die Anzeigevorrichtung blickt. Das wäre beispielsweise dann möglich, wenn der Patient flach liegt und dennoch auf dem Bildschirm etwas sehen soll.

Die Einrichtung zur Untersuchung des menschlichen Patienten 1 weist zumindest zwei, vorzugsweise mehrere unterschiedliche Messvorrichtungen auf, die jeweils Messdaten bezüglich des Zustandes des Patienten abgeben. Es ist weiters eine elektronische Auswerteinrichtung 5 vorgesehen, die in Abhängigkeit von aus zumindest zwei unterschiedlichen Messvorrichtungen stammenden Messdaten und deren Wechselbeziehung (Korrelation) Ausgangssignale ermittelt, die dann, wie die Figur 1 zeigt, beispielsweise über eine Leitung 13 einem für einen Arzt vorgesehenen Rechner (Arzt-PC 14) zuführbar sind. Die Ausgangssignale können auch als Steuerdaten für eines oder mehrere Therapiegeräte 15 über eine Leitung 16 zugeführt werden.

Außerdem können die Ausgangssignale in einem Bericht in digitaler oder gedruckter Form an den Arzt oder Therapeuten bzw. Patienten gegebenenfalls zusammen mit einem Therapievorschlag weitergegeben werden.

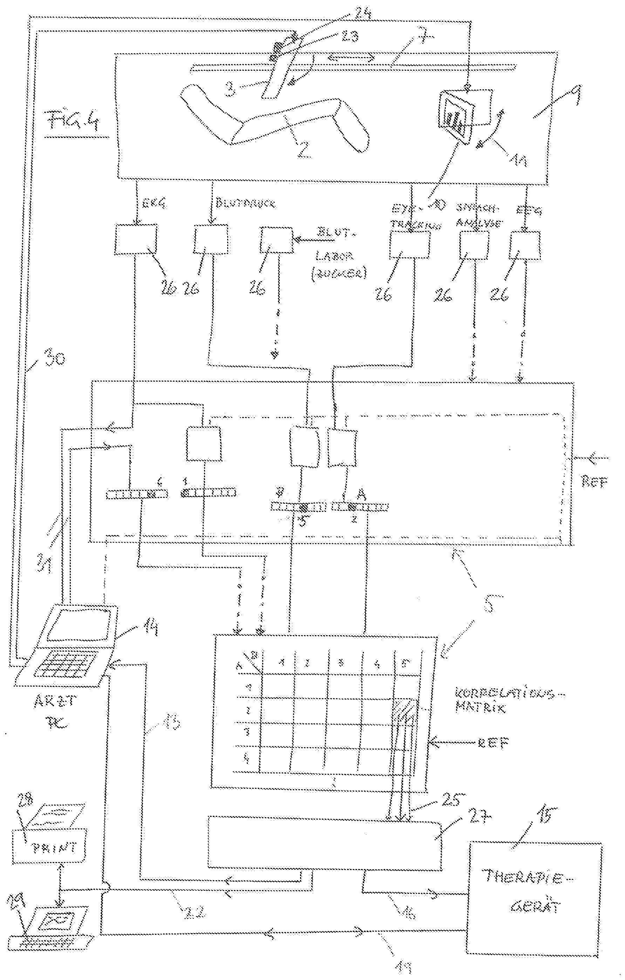

Weitere Details der Auswerteinrichtung werden weiter unten anhand der Figur 4 beschrieben.

Die Auswerteinrichtung kann jedenfalls auch teilweise dezentral arbeiten, wobei über eine schematisch dargestellte Leitung 17 und nicht näher dargestellte Kommunikationsmittel (beispielsweise über das Internet) auch ein an einem anderen Ort aufgestellter externer Server ansprechbar ist. In diesem externen Server kann einerseits Rechnerleistung erbracht werden, aber vor allem auch Referenzdaten abgespeichert werden, die es erlauben, die erfassten Messdaten des Patienten im Sinne einer Diagnose auszuwerten. Jedenfalls bleibt aber auch bei einer automatischen Therapie (Therapiegerät 15, Leitung 16) oder einem Therapievorschlag immer auch der Arzt beispielsweise über die Leitung 19 derjenige, der in den Therapievorschlag bzw. die automatische Therapie eingreifen kann.

Die Figur 2 zeigt einerseits verschiedene Position des als Sitzliege ausgebildeten Trägers für den Patienten, die je nach Untersuchungstyp motorisch eingestellt werden kann.

In Figur 2 links oben liegt der Patient auf dem Rücken. Es wird über ein Blutdruckmessgerät 4' mit Armmanschette und Messdatenaufbereitung der Blutdruck erfasst. Über ein EKG-Gerät 4" mit Elektroden 20 kann ein Elektrokardiogramm aufgezeichnet werden. Gleichzeitig oder danach kann ein Bild mit dem Thermoscanner 4 erzeugt werden, der in Richtung des Doppelpfeiles 7 linear bewegbar ist. Es ist aus der Figur 2 ersichtlich, dass die Tragvorrichtung 9 eine Aufnahme 12 (in optisch ansprechender elliptischer Form) aufweist, in der die diversen Messvorrichtungen bzw. deren Sensoren untergebracht sind.

Wie die einzelnen Abbildungen in Figur 2 zeigen, müssen nicht immer alle Messgeräte angeschlossen werden. Es können beispielsweise nur zwei Messvorrichtungen im Einsatz sein. Günstiger und bevorzugt ist allerdings vorgesehen, dass gleichzeitig oder knapp hintereinander mehrere Messungen vorgenommen werden. Dazu können insbesondere folgende Messvorrichtungen zum Einsatz kommen:
- eine Elektrokardiographie-Einrichtung (EKG),
- eine Einrichtung zur Feinstrommessung,
- eine Einrichtung zur Messung der Sauerstoffsättigung im Blut,
- eine Infrarot-Thermoscan-Einrichtung
- eine Einrichtung zum Blutdruckmessen,
- eine Einrichtung zur Messung der Herzratenvariabilität (HRV),
- eine Einrichtung zum Analysieren des Blutes,
- eine Einrichtung zur Frequenzanalyse von Organen,
- eine Einrichtung zur Messung von Photonen aus biologischem Gewebe,
- eine Einrichtung zur Magnet- und Impedanzmessung von biologischem Gewebe,
- eine Einrichtung zur Untersuchung der Haut und/oder der Zähne mittels bildgebender Erfassung,
- eine Einrichtung zur Untersuchung und Analyse der Atemluft,
- eine Ultraschall-Messvorrichtung,
- eine Elektroenzephalographie-Einrichtung (EEG),
- eine Elektromyographie-Einrichtung (EMG),
- eine Elektroneurographie-Einrichtung (ENG),
- eine vom Patienten bedienbare Eingabevorrichtung,
- eine Einrichtung zur Video-Erfassung des Ganges des Patienten.

Diese Einrichtungen sind im technischen Aufbau und Ihrer Anwendung im Einzelnen dem Fachmann bereits bekannt und brauchen hier nicht näher beschrieben zu werden. Neuartig an der Erfindung ist allerdings, dass diese Geräte in einer größeren Zahl und knapper hintereinander eingesetzt werden können, wenn ein Gesamtbild des Gesundheitszustandes des Patienten erwünscht ist.

Besonders bevorzugt ist ein Merkmal der Erfindung, dem neben den körperlichen Daten in den biologischen Screeningpositionen gemäß Figur 2 auch mentale Messdaten erfasst werden, die den psychischen Zustand des Patienten wiedergeben. Das ist schematisch in Figur 3 dargestellt. In Figur 3 links sieht man den Träger 2 in einer Sitzposition. Die Anzeigevorrichtung 3 in Form eines Touchscreens ist in Pfeilrichtung 8 heruntergeschwenkt und kann vom Patienten eingesehen werden. Während dem Patienten hier audiovisuelle Daten vorgespielt werden, wird über eine schematisch dargestellte EEG-Einrichtung 21 sowie über das Blutdruckmessgerät 4' Gehirnströme und Blutdruck erfasst. Damit kann man reaktive Daten erzielen, die über den psychischen Zustand des Patienten Aufschluss geben. Auch in einer Liegeposition, wie sie in Figur 3 rechts dargestellt ist, kann beispielsweise durch Beschallung oder durch Farbwechsel im Raum der Patient beeinflusst werden und festgestellt werden, welche Reaktionen dann erfasste Messdaten aufweisen. Der Übersichtlichkeit halber sind in den Figuren 2 und 3 nicht alle möglichen Anschlüsse von Messvorrichtungen gezeigt. Prinzipiell können alle oben in der Liste angegebenen Geräte teilweise, hintereinander und großteils auch gleichzeitig eingesetzt werden.

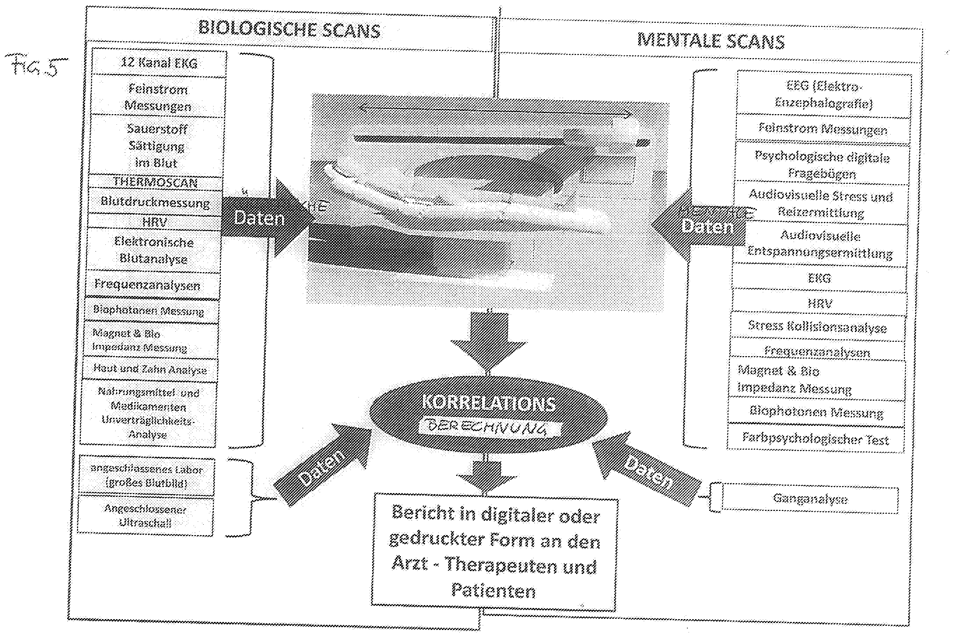

Besonders bevorzugt ist jedenfalls vorgesehen, dass der elektronischen Auswerteinrichtung 5 einerseits mentale Messdaten, die den psychischen Zustand des Patienten wiedergeben und andererseits körperliche Messdaten, die den körperlichen Zustand des Patienten wiedergeben, zugeführt werden, wobei die elektronische Auswerteinrichtung 5 aus den mentalen Messdaten und den körperlichen Messdaten und deren Wechselbeziehung (Korrelation) Ausgangssignale ermittelt, die dann über Leitungen 13, 16, 17, 22 weitere Funktionen erfüllen können, wie dies bereits anhand der Figur 1 kurz beschrieben worden ist und anhand der Figuren 4 und 5 noch näher beschrieben werden wird.

Bei dem in Figur 4 dargestellten Ausführungsbeispiel die Auswertung der Messdaten und die korrelative Ermittlung von Ausgangssignalen anhand eines vereinfachten Beispiels näher beschrieben.

Ganz oben in Figur 4 sieht man schematisch den Aufbau der mechanischen Komponenten der erfindungsgemäßen Einrichtung mit dem als Sitzliege ausgebildeten Träger 2. Der Patient ist aus Übersichtlichkeitsgründen nicht dargestellt. In einem ersten Schritt kann die Sitzliege 2 in eine Liegeposition gebracht werden und über hier nicht näher dargestellte, aber allgemein bekannte Messvorrichtungen bzw. Sensoren ein EKG erstellt, Blutdruck gemessen und auch in einem Blutlabor bestimmte Parameter, beispielsweise der Blutzucker festgestellt werden. Dabei handelt es sich im Wesentlichen um körperliche Messdaten, die dann der elektronischen Auswerteinrichtung 5 zugeführt werden.

In der in Figur 4 dargestellten Sitzstellung der Sitzliege 2 können dem Patienten über den Bildschirm 2 und einem nicht dargestellten Lautsprecher audiovisuelle Bilder vorgeführt werden. Seine Reaktion kann dann beispielsweise über eine Kamera 22 (Eyetracking) erfasst werden. Auch möglich ist eine Sprachanalyse, in dem der Patient angehalten wird, beispielsweise einen vorgegeben Text oder eine Antwort auf eine bildlich dargestellte Frage zu beantworten. Das Mikrophon 24 nimmt die Sprache auf und kann eine Sprachanalyse vornehmen. Auch ein Elektroenzephalogramm (EEG), wie es beispielsweise in der Figur 3 links dargestellt ist, kann erstellt werden. Schließlich ist es auch möglich, dem Patienten über den Bildschirm Fragen zu stellen, die er dann durch Eingabe in den Bildschirm beantwortet. All diese letztgenannten Messdaten sind mentale Messdaten, die den psychischen Zustand des Patienten wiedergeben.

In Figur 4 ist anhand eines vereinfachten Beispiels dargestellt, wie aus einer Korrelation eines Typs von körperlichen Messdaten (hier der Blutdruck B) mit einem Satz von mentalen Messdaten (hier Eyetracking A) zu definierten Ausgangssignalen auf den Ausgangsleitungen 25 der elektronischen Auswerteinrichtung 5 führt.

Ebenfalls vorhandene weitere Messdaten bzw. Auswertungen sind hier in strichpunktierten Linien dargestellt aber nicht weiter ausgeführt, um die Darstellung nicht zu kompliziert zu halten und das Wesen der im folgenden beschriebenen Korrelationsmatrix herauszuarbeiten.

Zunächst werden alle Messdaten in zugeordneten Speichereinrichtungen 26 zwischengespeichert, weil es im Allgemeinen nicht möglich ist, die körperlichen Daten auf den drei linken Leitungen in Figur 4 oben gleichzeitig mit den mentalen Messdaten auf den drei rechten Leitungen in Figur 4 gleichzeitig zu erfassen und damit zur Verfügung zu haben.

Über die Zwischenspeicher 26 können beliebige auch seriell hintereinander aufgenommene Messdaten gleichzeitig zur Verfügung stehen.

Am Beispiel des Satzes von körperlichen Messdaten Blutdruck B und des Satzes der mentalen Messdaten Eyetracking A, wird nun in der elektronischen Auswerteinrichtung zunächst eine zahlenmäßige Bewertung vorgenommen, wobei beim dargestellten Ausführungsbeispiel der Blutdruck mit der Zahl 5 und die Eyetracking-Messung mit der Zahl 2 bewertet wurde.

Diese zwei Zahlen kommen nun in eine Korrelationsmatrix, wobei jedes Feld der Korrelationsmatrix einem Wertepaar W, A einen eindeutigen Wert des Ausgangssignals zuordnet wobei dies, wie beim vorliegenden Beispiel dargestellt, auch mehrere Werte sein können, beispielsweise drei Ausgangswerte auf den Ausgangsleitungen 25. Beispielsweise kann es sich um drei Werte handeln, die ein Therapiegerät 15 über die Sammelleitung 16 ansteuern. Es kann aber auch in der Ausgabeeinheit 27 zu einer Aufbereitung der Werte für eine Ausgabe auf einen Drucker 28 oder einen Bildschirm 29 kommen. Auch eine Aufbereitung für den Arzt WC 14 und Übermittlung der Daten der Überleitung 13 ist möglich.

Der Arzt kann dann entscheiden, ob er die Daten insgesamt oder teilweise mit dem Patienten teilen will und gegebenenfalls diese auf dem Zusatzbildschirm 10 darstellen. Der Arzt kann überhaupt über die Leitung 30 den gesamten Messdatenerfassungsprozess, insbesondere den Scanprozess steuern. Der Arzt-PC 14 kann auch über drahtgebundene oder drahtlose Verbindungen mit anderen Komponenten, insbesondere der elektronischen Auswerteinrichtung 5 kommunizieren. Er kann auch über das Internet mit dezentralen externen Servern verbunden sein. Dasselbe gilt für die elektronische Auswerteinrichtung, die selbst direkt über das Internet mit Referenzdaten REF aus einer externen Quelle versorgt werden kann. Das ist insbesondere auch über das Internet möglich.

Diese Referenzdaten erlauben es unter anderen, die geschilderte zahlenmäßige Bewertung der Messsignale, hier Blutdruck und Eyetracking B, A vorzunehmen und einzuordnen.

Weiters ist es auch möglich, dass die Werte in der Korrelationsmatrix über einen externen Referenzeingang REF bestimmt werden. Es ist aber auch möglich, dass in der Korrelationsmatrix eine Funktion hinterlegt ist, die aus den Zahlen ausgedrückten Messdaten und einem bekannten funktionellen Zusammenhang das entsprechende Matrix-Element oder den Satz von Matrix-Elementen ermittelt und über die Ausgabeleitungen 25 ausgibt.

Beim dargestellten Ausführungsbeispiel ist die Matrix lediglich zweidimensional, weil es sich nur um zwei dargestellte herausgegriffene Messdaten gehandelt hat.

Die Matrix wird bei mehreren Messdaten aber selbstverständlich dreidimensional ausfallen (was dann aber grafisch nicht mehr dargestellt werden kann). In jedem Fall können hier aber gemäß einem bevorzugten Merkmal der Erfindung körperliche Messedaten bzw. deren zugeordnete Zahlenwerte und mentale Messdaten bzw. deren zugeordnete Zahlenwerte miteinander korreliert werden und zu Ausgangssignalen verarbeitet werden.

Anhand der Verarbeitung der Messsignale des EKGs sei noch dargestellt, dass eine Messung (hier EKG) unter anderem mehrere Teilergebnisse bringt, die dann gesondert als Messdatenwerte zahlenmäßig zur Verfügung stehen.

Beispielsweise lässt sich die Lage bestimmter Abschnitte des EKGs leichter automatisch ermitteln. Das ist im vorliegenden Beispiel in Figur 4 Mitte links dargestellt, wo die zahlenmäßige Bewertung zum Wert 1 geführt hat.

Andere Eigenschaften des EKGs erlauben manchmal keine automatische Bewertung. Hier wird der Arzt über die "Beurteilungsleitungen 31" eingeschaltet. Er sieht dann am PC 14 das EKG und kann es zahlenmäßig beurteilen, beispielsweise mit der Zahl 6, wie es im vorliegenden Beispiel dargestellt ist. Anschließend kann wieder eine vollständig automatisierte Korrelation vorgenommen werden.

Erfindungsgemäß ist in einer bevorzugten Ausführungsform vorgesehen, dass die mentalen Messdaten einerseits und die körperlichen Messdaten andererseits zumindest teilweise aus unterschiedlichen Messvorrichtungen stammen, die für das jeweilige Aufgabengebiet besonders adaptiert sind. Es ist aber durchaus möglich, dass ein- und dieselbe Messvorrichtung für beide Zwecke, also zur Ermittlung von körperlichen Messdaten und zur Ermittlung von mentalen Messdaten verwendet wird. Dann ist es besonders günstig, wenn eine Einrichtung vorzugsweise zur nicht invasiven und berührungslosen Beeinflussung des Patienten vorgesehen ist und zumindest eine Messvorrichtung die Reaktion des Patienten erfasst und davon abhängige reaktive Messdaten ausgibt, die dann als mentale Messdaten verwendet werden können.

Beispielsweise kann der Patient über Bildfolgen oder Videos beeinflusst werden und die Reaktion am EKG angesehen werden, um mentale Messdaten zu erhalten. Im Ruhezustand kann dasselbe EKG körperliche Messdaten erfassen.

Zur Erfassung von körperlichen Messdaten eignen sich besonders folgende Messvorrichtungen:
- eine Elektrokardiographie-Einrichtung (EKG),
- eine Einrichtung zur Feinstrommessung,
- eine Einrichtung zur Messung der Sauerstoffsättigung im Blut,
- eine Infrarot-Thermoscan-Einrichtung
- eine Einrichtung zum Blutdruckmessen,
- eine Einrichtung zur Messung der Herzratenvariabilität (HRV),
- eine Einrichtung zum Analysieren des Blutes,
- eine Einrichtung zur Frequenzanalyse von Organen,
- eine Einrichtung zur Messung von Photonen aus biologischem Gewebe,
- eine Einrichtung zur Magnet- und Impedanzmessung von biologischem Gewebe,
- eine Einrichtung zur Untersuchung der Haut und/oder der Zähne mittels bildgebender Erfassung,
- eine Einrichtung zur Untersuchung und Analyse der Atemluft,
- eine Ultraschall-Messvorrichtung,
- eine Elektromyographie- Einrichtung (EMG).

Zur Erfassung von mentalen Messdaten eignen sich besonders folgende Messvorrichtungen:
- eine Elektrokardiographie-Einrichtung (EKG),
- eine Einrichtung zur Feinstrommessung,
- eine Einrichtung zur Messung der Herzratenvariabilität (HRV),
- eine Einrichtung zur Frequenzanalyse von Organen,
- eine Einrichtung zur Messung von Photonen aus biologischem Gewebe,
- eine Einrichtung zur Magnet- und Impedanzmessung von biologischem Gewebe,
- eine Elektroenzephalographie-Einrichtung (EEG),
- eine Elektroneurographie-Einrichtung (ENG),
- eine vom Patienten bedienbare Eingabevorrichtung,
- eine Einrichtung zur Video-Erfassung des Ganges des Patienten.

Günstigerweise wird man als mentale vor allem reaktive Messdaten verwenden, vorzugsweise nur solche.

Ein wesentlicher Vorteil der erfindungsgemäßen Einrichtung besteht darin, dass sie in der Lage ist, mentale und körperliche Messdaten miteinander zu verknüpfen und in Abhängigkeit von dieser Korrelation Ausgangssignale zur Verfügung stellt. Damit lässt sich ein umfassendes Gesamtbild des Gesundheitszustandes des Patienten erzielen und dieses auch an den Arzt, Therapeuten und Patienten kommunizieren. Außerdem ist es möglich, diese Daten als Steuersignale für angeschlossene Therapievorrichtungen zu verwenden.

In Figur 5 sind nochmals im Überblick die wesentlichen biologischen Scans zur Ermittlung von körperlichen Messdaten links und die mentalen Scans zur Ermittlung von mentalen Messdaten rechts im Überblick dargestellt.

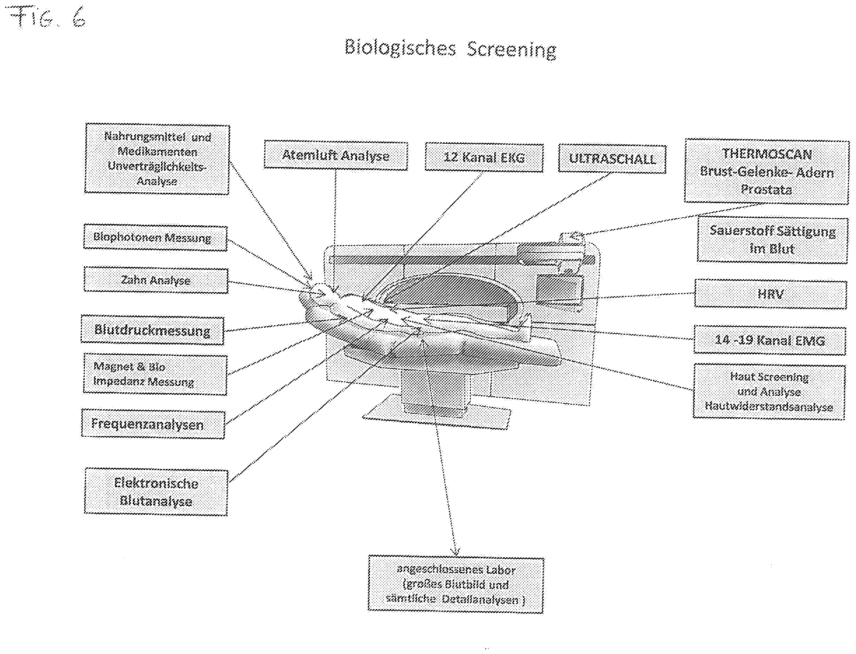

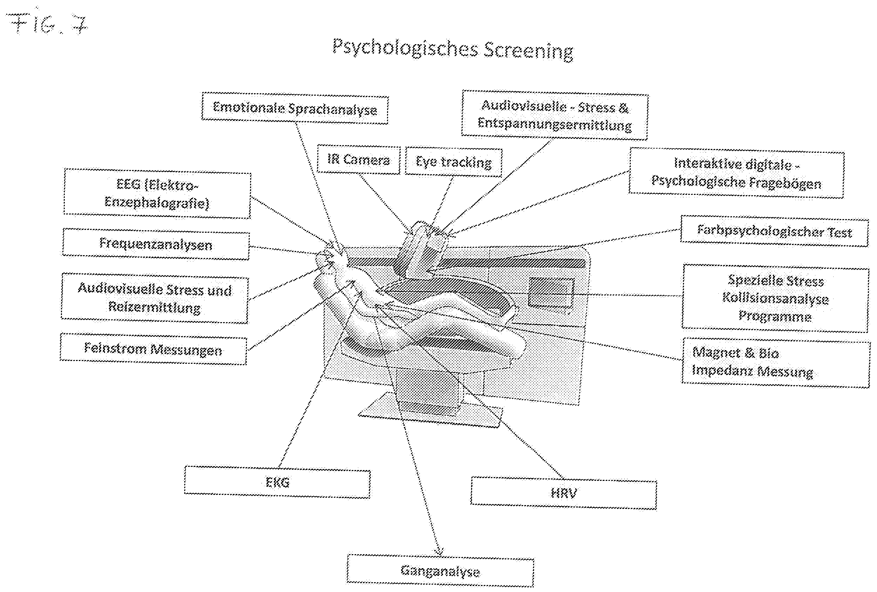

Die Figuren 6 und 7 zeigen in gesonderten Darstellungen das biologische Screening zum Ermitteln von körperlichen Messdaten und das psychologische Screening zum Ermitteln von mentalen Messdaten, die den psychischen Zustand des Patienten wiedergeben. Insgesamt ist eine technische Verarbeitung aller gleichzeitig und in kurzer Zeit ermittelten biologischen und psychologischen Gesundheitsparametern samt Korrelationsüberprüfung möglich. Ausgaben erfolgen an elektrotechnische und digitale Geräte, Anweisungen an die verschieden angeschlossenen therapeutischen Equipments gegeben werden.

Die "Full Body Screening"-Anlage ist in der Lage, die in ganz kurzer Zeit ermittelten und analysierten Patienten Daten aus seinem biologischen und mentalen bzw. psychologischen Bereich nach der erfolgten Korrelationsberechnung, die auch über einen zentralen Server erfolgen kann, in eine technische Maschinensprache umzusetzen und an die verschiedensten therapeutischen Equipments automatisiert zu übermitteln.

Dabei werden sowohl audiovisuelle- als auch elektrotechnische Signale an die verschiedenen Equipments transferiert. Mit "Wenn Dann" Befehlen werden die angeschlossenen Equipments angesteuert, wobei der Arzt oder Therapeut jederzeit die Möglichkeit hat in den Therapieverlauf einzugreifen, sollte er das für erforderlich halten.

Die verschiedenen Equipments wiederum senden die Ergebnisse nach durchgeführten Therapien zurück zum zentralen Server bzw. zur Auswerteinrichtung Diese Ergebnisprüfung wird durch in den Equipments eingebauten Sensoren wie beispielweise HRV - EEG - EKG - Magnetimpedanzmessungen - Feinstrom Messungen - Bewegungssensoren - Blutdruck und Blutsauerstoff Messungen - Frequenz Messungen usw. ermittelt. Damit erhält der Arzt zusätzlich die Informationen, wie die Therapien angeschlagen haben.

Die Daten können auch "just in time" an ein zwischengeschaltetes telemedizinisches Zentrum gesendet werden und dort von Spezialisten und medizinischem Fachpersonal gecheckt werden. In der Patienten-Datenbank im Zentralserver wird bevorzugt nach der Behandlung ein weiterer Korrelationslauf durchgeführt, um die Ergebnisse der Therapien mit den Ausgangsdaten abgleichen zu können und gegebenenfalls die Therapien adaptieren zu können.

Die Screening-Anlage ermittelt den Gesundheitsstatus:
- in einer bisher noch nie dagewesenen kurzen Zeit
- und in einer hohen Dichte an Daten und Informationen sowohl aus dem biologischen als auch aus dem psychologischen bzw. dem mentalen Bereich inklusive dem Verhaltens- relevanten Bereich. Und das alles "non-invasiv" und bevorzugt ohne Strahlenbelastung.

Die verschiedenen Screening Ergebnisse werden in der Folge noch im Detail beschrieben. Dadurch können die Untersuchungszeiten von mehreren Stunden und sehr oft von Tagen, insbesondere wenn verschiedenen Spezialisten bzw. Fachärzte aufgesucht werden müssen, eingespart werden. Der Screening Vorgang mit erfindungsgemäßer Anlage dauert in den meisten Fällen nur wenige Minuten. Bei schwerwiegendem Problem bis maximal 1 Stunde. Wertvolle Lebenszeit - verlorene Arbeits- oder Freizeit kann dadurch gewonnen werden.

Die Screening-Anlage ermittelt den Gesundheitsstatus der Menschen in einer noch nie dagewesenen holistischen Art und Weise. Dadurch werden bestehende oder erst entstehende Krankheiten aufgezeigt die mit einer normalen Untersuchung kaum und schon gar nicht mit diesem geringen Zeiteinsatz_entdeckt werden würden.

Die Screening-Anlage ist in der Lage nicht nur die Symptome sondern auch viele Ursachen und Fehlverhalten die zu einer Krankheit führen, aufzuspüren und gezielten Therapien zuzuführen. Die meisten derzeitigen Analysen konzentrieren sich auf die Diagnose von erkennbaren Symptomen. Die erfindungsgemäße Anlage konzentriert sich neben einer hohen Diagnose-Genauigkeit vor allem auch auf die Ursachenermittlung, wobei einige Therapien von der Anlage direkt durchgeführt werden können.

Ein weiterer Vorteil der erfindungsgemäßen Anlage ist der Umstand, dass diese Anlage alle ermittelten Paramater mit zahlreichen Vergleichsdaten abgleichen kann und über einen speziellen Algorithmus, insbesondere Matrix-Berechnung, herausfindet, welchen zukünftigen Verlauf der ermittelte Vitalitäts- bzw. Gesundheits- oder Krankheitsstatus nehmen wird. Dabei kann simuliert werden, wie sich der Zustand des Menschen verändert, sofern er gleich weiterlebt bzw. was passiert, wenn er seine Lebensweise ändert.

Darüber hinaus kann simuliert und dargestellt werden, welchen Einfluss verschiedene Medikamente und Naturheil- und Nahrungsmittel auf den untersuchten Menschen haben werden.

Damit kann die Lebensqualität verbessert und das Leben verlängert werden. Vor allem können eventuell entstehende Krankheiten vermieden und bestehenden Krankheiten eliminiert werden.

Die Screening-Anlage kann auch Nahrungsmittel-Unverträglichkeiten in einer in dieser Kombination noch nie dagewesenen Präzision erkennen. Dabei wird über ein Ganzkörper-Scanning über Photonen aus Gewebe und Magnetfeld und Energiedichtemessungen die generelle Unverträglichkeit geprüft. Und parallel dazu wird über Bluttests und einem Atemluft Test die Unverträglichkeit geprüft. Ein spezielle HRV (Herzratenvariabilität) Analyse vertieft und bestätigt das Testergebnis. Damit ergibt sich eine Analyse- und Diagnose-Qualität, die sonst nur mit sehr vielen verschiedenen Untersuchungen meist bei verschiedenen Spezialisten möglich ist.

Damit können die Essgewohnheiten der Menschen so abgestimmt werden, dass diese die optimale Ernährung genießen können, ohne mit unverträglichen Lebensmitteln den Körper bzw. die Gesundheit zu belasten.

Die Anlage ist weiters in der Lage, Medikamente-Unverträglichkeiten festzustellen und über die vielen in der Anlage integrierten Vergleichsparameter verträgliche Alternativen vorzuschlagen. Diese Alternativen können zusätzlich durch weitere Screenings auf Ihre Verträglichkeit verifiziert werden.

Der Mensch bekommt genau jene Medikation, die für ihn ideal ist. Unverträgliche Medikamente werden erkannt und können durch verträgliche Medikamente und Behandlungsmethoden ersetzt werden.

Insgesamt ist die Anlage in der Lage, nicht nur exzellente Analysen und Diagnosen zu erstellen, sondern auch, und das ist ebenfalls weltweit einzigartig, diese Analysen und Diagnosen mit verschiedenen Methoden direkt am Gerät zu therapieren oder Therapien automatisiert vorzuschlagen.

Der Nutzer der Anlage spart sich wiederum Zeit und verschiedene Wege zu diversen Spezialisten und kann sofort umfassend und nach modernsten Erkenntnissen therapiert werden.

Die über die Anlage ermittelten Daten und Fakten können in Echtzeit an ein telemedizinisches Zentrum weitergeleitet werden. Das Analyse- und Diagnose-Programm der Screening Anlage stellt nicht nur all Gesundheits- bzw. Krankheitsfakten dar Fakten dar, sondern schlägt auch gleichzeitig die bestmöglichen Behandlungsmethoden vor. Im Telemedizin-Zentrum oder vor Ort neben der Anlage kontrolliert ein Ärzte-Team diese Daten und Fakten und implementiert eine finale Empfehlung in den Statusbericht der erfindungsgemäßen Anlage.

Der überprüfte Bericht geht nun an den behandelnden Arzt bzw. an das medizinische Fachpersonal vor Ort, die ja mit dem Patienten bzw. dem Gast in direkter Verbindung stehen. Mit diesem kontrollierten und bei Bedarf durch das Telemedizinzentrum adaptierten Bericht bekommt das medizinische Fachpersonal eine perfekte Information, um den Nutzer vor Ort zu betreuen.

Durch die hohe Qualität der ermittelten Daten und Fakten sowie der durch die Anlage erstellten Diagnosen und Behandlungsempfehlungen sowie durch die übergeordnete Kontrolle durch das Telemedizin-Zentrum oder einen Arzt vor Ort erhält das medizinische Fachpersonal die bestmöglichen Unterlagen, um den Patienten/Gast zu behandeln und zu betreuen.

Gerade für noch junge und etwas unerfahrenere Ärzte ist diese Information extrem wichtig, um Fehldiagnosen und falsche Behandlungen möglichst zu verhindern. Aber auch ein noch so erfahrender Arzt ist erstaunt und extrem positiv überrascht, welch wertvolle Informationen er über das erfindungsgemäße System erhalten kann. Auch der beste Arzt kann niemals diese ganzen Informationen in derart kurzer Zeit ermitteln.

Die Analysen, die Diagnosen und die Behandlungsempfehlungen, die über die Anlage an die Patienten übergeben werden, sind völlig neuartig, insbesondere die Tatsache. dass all diese Ergebnisse der Analysen, der Diagnosen, der Risiko-Parameter und auch die empfohlenen Behandlungen in einer für jeden Laien verständlicher Sprache übermittelt werden können. Dabei wird diese Informationen mit anschaulichen bzw. erklärenden Grafiken und Videos unterstützt und vervollständigt.

Die gesamte Information als auch sämtliche medizinischen Fachausdrücke kann das System automatisch in die jeweilige Landessprache des Patienten/Gastes übersetzen. Der Arzt bzw. das medizinische Fachpersonal wiederum bekommt alle Daten in einem bekannten medizinischen Format zur Verfügung gestellt, wobei der Umfang der Information auf Grund Informationsdichte und der Daten-Qualität einzigartig ist.

Die meisten Analysen, die der Mensch heute bekommt, sind gespickt mit Fachausdrücken, die nur ein Arzt interpretieren kann. Damit ist eine enorme Verunsicherung vorprogrammiert.

Der Nutzer der Anlage bekommt sämtlich Informationen in einer verständlichen, zum Teil auch grafischen und bildhaften animierten Weise, in seiner Landessprache erklärt und kann diese Information bestens verstehen und verwerten.

Der Mediziner bekommt wesentlich mehr Informationen über den Patienten/Gast und das in einer ganz kurzen Zeit und kann damit seine Behandlung wesentlich zielgerichteter ausrichten. Durch die Zeitersparnis ergibt sich für den Mediziner darüber hinaus auch noch ein ganz wesentlich wirtschaftlicher Vorteil.

Folgende Screenings, die nicht Gegenstand der vorliegenden Erfindung sind, können im Wesentlichen synchron während der Untersuchung in ganz kurzer Zeit durchgeführt und umfassende Daten ermitteln, die dann unmittelbar darauf in eine Behandlungs-Empfehlung an das medizinische Personal übermittelt werden.

Allgemeine Gesundheits- bzw. Vitalitäts-Status-Ermittlung
- Erhebung des Vitalitätsstatus im Vergleich zu Referenzgruppen.
- Unter Einsatz von Organ-Frequenz-Analysen
- Unter Einsatz von (HRV) Herzraten-Variabilitäts-Checks.
- Unter Einsatz von Photonen-Analysen aus menschlichem Gewebe.
- Unter Einsatz von Energie Dicht Messungen der einzelnen Organe.
- Ermittlung von Entzündungen im Körper
- Ermittlung von Onkologischen Problemen.
- Blutstatus Ermittlung
- Zahnstatus Ermittlung
- Atemluft-Status
- Hautstatus
- Entzündungsstatus
- Augenstatus
- Check sämtlicher Organe in Bezug auf eventueller Krankheiten und Risikofaktoren.
- Soll Ist Vergleiche in Bezug auf Mineral Mankos
- Ermittlung von Medikamenten Unverträglichkeiten
- Ermittlung von Lebensmittel Unverträglichkeiten
- EKG
- Sauerstoff Sättigung des Blutes.
- Erhebung des mentalen bzw. psychologischen Gesundheits- Status

Unmittelbar nach Erhebung des allgemeinen Gesundheits- und Vitalitäts-Status werden, sofern bei einzelnen Organen Irritationen erkannt wurden, die betroffenen Organe speziell gescannt und bei Bedarf ganz spezielle Therapie-Empfehlungen abgegeben.

Das gilt auch für den mentalen bzw. psychologischen Gesundheitsstatus sowie der Haut:
- Erhebung des Haut Durchblutungs- Status über hochsensible Thermografie Ermittlung von Entzündungen
- **PH** Wert Ermittlung der Haut in verschiedenen Hautzonen.
- Ermittlung von Hautirritationen
- Hochauflösende Aufnahme von Muttermalen und Hautirritationen die auf einen eventuellen Hautkrebs hinweisen und Abgleich mit tausenden Referenzbildern
- Ermittlung von Haut Allergien.

Zähne:
- Erhebung des Zahnstatus über Biophotonen und Energiedichte Messung
- Aufnahme des optischen Zahnstatus
- Kontrolle über Entzündungsmarker

Bezüglich Brustuntersuchungen ist beispielsweise folgendes möglich:
- Erhebung der Brustgesundheit über Thermografisches Screening mit Bildgebender Darstellung ohne jegliche Strahlenbelastung.
- Sichtbarmachung von Entzündungen und Brustirritationen auch in einem sehr frühen Stadium
- Doppel Check über Krebsmarker aus der Blutuntersuchung.
- Check des gesamten Bereichs über Biophotonen - Energie Dichte Messungen - HRV Messungen

Bezüglich Gelenken:
- Screening der Gelenke um Irritationen - Abnützungen - und Entzündungen feststellen zu können.

Dabei werden HRV Messungen, Photonen-Messungen, Energie-Dichte-Messungen und Thermosensoren mit Bildgebender Darstellung eingesetzt.

Bezüglich Männergesundheit:
- Der gesamte Männergesundheitsbereich wird analysiert. Dabei wird die Prostata - der Blasenbereich, die Potenz und alles was mit der Männergesundheit zusammenhängt, gescreent und diagnostiziert - sowie Behandlungen vorgeschlagen.

Bezüglich Frauengesundheit:
- Der gesamte Frauengesundheitsbereich wird analysiert. Dabei wird die Gebärmutter - der Blasenbereich (Inkontinenz) und alles was mit der Frauengesundheit zusammenhängt, gescreent und diagnostiziert - sowie Behandlungen vorgeschlagen.

Bezüglich Organen im Allgemeinen:
- Sofern bei den einzelnen Organen bei der Vitalstatus-Erhebung Auffälligkeiten entdeckt wurden, wird jedes betroffene Organ detailliert gescreent und diagnostiziert und eine Behandlungsempfehlung ausgedruckt.

Dies ist auch der Grund, warum die normalerweise sehr kurze Diagnosezeit sich auf bis zu eine Stunde verlängern kann. Wobei selbst eine Stunde nur ein Bruchteil des Zeitbedarfs ausmacht, den man benötigt, um alle Organe bei den verschiedenen Fachärzten untersuchen zu lassen.

Zur mentalen und psychosozialen Gesundheit kann beispielsweise folgendes ermittelt werden:
- Mit speziellen Screening-Verfahren, die über bewegte Bilder (Kurzfilme) über Sprachausgaben über Texteingaben und parallel über Photonen und Energie-Dichte-Messungen über Reaktionsanalysen - (Schweiß und Körpertemperatur Messungen) sowie über HRV Kontrollmessungen erfolgen.
- Auch werden die möglichen Erregungs- und Entspannungszustände gemessen.
- Es werden die höchsten Stressfaktoren und die besten Entspannungsmethoden ermittelt und in einem automatisiert erstellten Therapiepaket dem Patienten/ Gast und dem medizinischen Fachpersonal übermittelt. Darüber hinaus wird ein personalisiertes Farbprofil erstellt und eine individuelle Farbtherapie automatisiert ausgearbeitet, die wiederum mit einer speziellen Aromatherapie Empfehlung verknüpft ist.
- Vor allem werden die wesentlichen Beziehungskollisions-Faktoren ermittelt, die zu einer Stressbelastung führen.

## Patentansprüche

1. Einrichtung zur Untersuchung eines menschlichen Patienten (1) mit einem Träger (2) für den Patienten (1), mindestens einer - insbesondere als Bildschirm ausgebildeten - Anzeigevorrichtung (3), die von dem auf dem Träger (2) befindlichen Patienten (1) einsehbar ist, mindestens zwei Messvorrichtungen (4) zum Erfassen von Messdaten des Patienten (1), die Messdaten bezüglich des Zustands des Patienten ausgeben, sowie einer elektronischen Auswerteinrichtung (5), die mit der Anzeigevorrichtung (3) und der Messvorrichtung (4) in Verbindung steht, und die dazu ausgelegt ist, in Abhängigkeit von aus zumindest zwei unterschiedlichen Messvorrichtungen stammenden Messdaten und deren Wechselbeziehung Ausgangssignale zu ermitteln, wobei zumindest eine Anzeigevorrichtung und zumindest ein Sensor (4a) einer Messvorrichtung (4) gegenüber dem Träger (2) für den Patienten (1) beweglich gelagert ist, **dadurch gekennzeichnet, dass** die zumindest eine Anzeigevorrichtung (3) und der zumindest eine Sensor (4a) einer Messvorrichtung (4) motorisch bewegbar an einer Tragvorrichtung (9) gelagert ist, und dass die zumindest eine Messvorrichtung (4) einen medizinischen Thermoscanner umfasst.

2. Einrichtung nach Anspruch 1, **dadurch gekennzeichnet, dass** zumindest eine Anzeigevorrichtung und zumindest ein Sensor (4a) einer Messvorrichtung (4) auf einem gemeinsamen Halter (6), insbesondere einem verfahrbar gelagerten Schlitten, bewegbar gelagert sind.

3. Einrichtung nach Anspruch 2, **dadurch gekennzeichnet, dass** der Halter (6) einen verfahrbaren Basisteil und einen an diesem verschwenkbar gelagertern Schwenkteil aufweist, der die zumindest eine Anzeigevorrichtung (3) und den zumindest einen Sensor (4a) einer Messvorrichtung (4) trägt.

4. Einrichtung nach einem der Ansprüche 1 bis 3, **dadurch gekennzeichnet, dass** der Anzeigevorrichtung eine vom Patienten bedienbare Eingabevorrichtung, insbesondere in Form eines Touch-Screens, zugeordnet ist.

5. Einrichtung nach einem der Ansprüche 1 bis 4, **dadurch gekennzeichnet, dass** die zumindest eine Messvorrichtung (4) eine - insbesondere zum Eye-Tracking geeignete - Kamera und/oder ein Mikrofon zum Aufnehmen von Lauten des Patienten umfasst.

6. Einrichtung nach einem der Ansprüche 1 bis 5, **dadurch gekennzeichnet, dass** an einer - vorzugsweise mit einem Gehäuse versehenen - Tragvorrichtung (9), an der die Anzeigevorrichtung beweglich gelagert ist, eine weitere Anzeigevorrichtung (10) - vorzugsweise starr oder schwenkbar - gelagert ist.

7. Einrichtung nach einem der Ansprüche 1 bis 6, **dadurch gekennzeichnet, dass** der Träger (2) für den Patienten (1) als mehrteilige Sitzliege ausgebildet ist, deren Teile in ihrer Lage - vorzugsweise motorisch - verstellbar sind, wobei die Sitzliege vorzugsweise dreiteilig mit einem Brustteil, einem Mittelteil und einem Fußteil ausgebildet ist.

8. Einrichtung nach einem der Ansprüche 1 bis 7, **dadurch gekennzeichnet, dass** die Messvorrichtungen zumindest zwei der folgenden Messvorrichtungen umfasst:
- eine Elektrokardiographie-Einrichtung (EKG),
- eine Einrichtung zur Feinstrommessung,
- eine Einrichtung zur Messung der Sauerstoffsättigung im Blut,
- eine Infrarot-Thermoscan-Einrichtung
- eine Einrichtung zum Blutdruckmessen,
- eine Einrichtung zur Messung der Herzratenvariabilität (HRV),
- eine Einrichtung zum Analysieren des Blutes,
- eine Einrichtung zur Frequenzanalyse von Organen,
- eine Einrichtung zur Messung von Photonen aus biologischem Gewebe,
- eine Einrichtung zur Magnet- und Impedanzmessung von biologischem Gewebe,
- eine Einrichtung zur Untersuchung der Haut und/oder der Zähne mittels bildgebender Erfassung,
- eine Einrichtung zur Untersuchung und Analyse der Atemluft,
- eine Ultraschall-Messvorrichtung,
- eine Elektroenzephalographie-Einrichtung (EEG),
- eine Elekromyographie-Einrichtung (EMG),
- eine Elektroneurographie-Einrichtung (ENG),
- eine vom Patienten bedienbare Eingabevorrichtung,
- eine Einrichtung zur Video-Erfassung des Ganges des Patienten.

9. Einrichtung nach einem der Ansprüche 1 bis 8, **dadurch gekennzeichnet, dass** der elektronischen Auswerteinrichtung einerseits mentale Messdaten, die den psychischen Zustand des Patienten wiedergeben, und andererseits körperliche Messdaten, die den körperlichen Zustand des Patienten wiedergeben, zugeführt werden, wobei die elektronische Auswerteinrichtung (5) dazu ausgelegt ist, aus den mentalen Messdaten und den körperlichen Messdaten und deren Wechselbeziehung Ausgangssignale zu ermittelt.

10. Einrichtung nach Anspruch 9, **dadurch gekennzeichnet, dass** die mentalen Messdaten einerseits und die körperlichen Messdaten andererseits zumindest teilweise aus unterschiedlichen Messvorrichtungen stammen.

11. Einrichtung nach Anspruch 10, **dadurch gekennzeichnet, dass** die körperlichen Messdaten aus zumindest einer der folgenden Messvorrichtungen stammen:
- eine Elektrokardiographie-Einrichtung (EKG),
- eine Einrichtung zur Feinstrommessung,
- eine Einrichtung zur Messung der Sauerstoffsättigung im Blut,
- eine Infrarot-Thermoscan-Einrichtung
- eine Einrichtung zum Blutdruckmessen,
- eine Einrichtung zur Messung der Herzratenvariabilität (HRV),
- eine Einrichtung zum Analysieren des Blutes,
- eine Einrichtung zur Frequenzanalyse von Organen,
- eine Einrichtung zur Messung von Photonen aus biologischem Gewebe,
- eine Einrichtung zur Magnet- und Impedanzmessung von biologischem Gewebe,
- eine Einrichtung zur Untersuchung der Haut und/oder der Zähne mittels bildgebender Erfassung,
- eine Einrichtung zur Untersuchung und Analyse der Atemluft,
- eine Ultraschall-Messvorrichtung,
- eine Elekromyographie-Einrichtung (EMG),
wobei die mentalen Messdaten aus zumindest einer der folgenden Messvorrichtungen stammen:
- eine Elektrokardiographie-Einrichtung (EKG),
- eine Einrichtung zur Feinstrommessung,
- eine Einrichtung zur Messung der Herzratenvariabilität (HRV),
- eine Einrichtung zur Frequenzanalyse von Organen,
- eine Einrichtung zur Messung von Photonen aus biologischem Gewebe,
- eine Einrichtung zur Magnet- und Impedanzmessung von biologischem Gewebe,
- eine Elektroenzephalographie-Einrichtung (EEG),
- eine Elektroneurographie-Einrichtung (ENG),
- eine vom Patienten bedienbare Eingabevorrichtung,
- eine Einrichtung zur Video-Erfassung des Ganges des Patienten.

12. Einrichtung nach einem der Ansprüche 1 bis 11, **dadurch gekennzeichnet, dass** eine Einrichtung zur - vorzugsweise nicht invasiven und berührungslosen - Beeinflussung des Patienten vorgesehen ist, die vorzugsweise einen Bildschirm (3) und/oder einen Lautsprecher umfasst, wobei zumindest eine Messvorrichtung dazu ausgelegt ist, die Reaktion des Patienten zu erfassen und davon abhängige reaktive Messdaten auszugeben.

13. Einrichtung nach Anspruch 12, **dadurch gekennzeichnet, dass** sie eine vom Patienten bedienbare Eingabevorrichtung, insbesondere in Form eines Touch-Screens (3), aufweist, wobei die reaktiven Messdaten den in die Eingabevorrichtung eingegebenen Eingabedaten entsprechen.

14. Einrichtung nach Anspruch 12 oder 13, **dadurch gekennzeichnet, dass** die Einrichtung so ausgelegt ist, dass als mentale Messdaten die reaktiven Messdaten, und vorzugsweise nur diese, verwendet werden.

15. Einrichtung nach einem der Ansprüche 1 bis 14, **dadurch gekennzeichnet, dass** zumindest eine Therapievorrichtung (15) vorgesehen ist, der mindestens ein Teil der Ausgangssignale der elektronischen Auswerteinrichtung (5) als Steuersignale zugeführt werden, in deren Abhängigkeit die Therapievorrichtung unterschiedlich arbeitet.

16. Einrichtung nach einem der Ansprüche 1 bis 15, **dadurch gekennzeichnet, dass** zumindest eine Anzeigevorrichtung (14) für den Arzt, zumindest eine Anzeigevorrichtung (10) für den Patienten und/oder zumindest eine Druckvorrichtung (28) vorgesehen sind (ist), denen jeweils Ausgangssignale aus der elektronischen Auswerteinrichtung (5) zur graphisch aufbereiteten Anzeige und/oder Druck zugeführt werden.

## Claims

1. An apparatus for examining a human patient (1) with a support (2) for the patient (1), at least one display device (3) - especially designed as a screen - which can be viewed by the patient (1) situated on the support (2), at least two measuring devices (4) for recording measurement data from the patient (1), which display measurement data relating to the condition of the patient, and an electronic evaluation device (5), which is connected to the display device (3) and the measuring device (4), and which is designed to determine output signals based on measurement data obtained from at least two different measuring devices and the correlation thereof, wherein at least one display device and at least one sensor (4a) of a measuring device (4) are mounted movably relative to the support (2) for the patient (1), **characterized in that** the at least one display device (3) and the at least one sensor (4a) of a measuring device (4) is mounted on a support device(9) so as to be movable via a motor, and that the at least one measuring device (4) comprises a medical thermoscanner.

2. The apparatus according to claim 1, **characterized in that** at least one display device and at least one sensor (4a) of a measuring device (4) are movably mounted on a common holder (6), especially a maneuverably supported carriage.

3. The apparatus according to claim 2, **characterized in that** the mount (6) has a movable base portion and a pivot part pivotably mounted thereon, which carries the at least one display device (3) and the at least one sensor (4a) of a measuring device (4).

4. The apparatus according to one of claims 1 to 3, **characterized in that** the display device is assigned to an input device operable by the patient, in particular in the form of a touch screen.

5. The apparatus according to one of claims 1 to 4, **characterized in that** the at least one measuring device (4) has a camera - particularly one suitable for eye tracking - and/or a microphone for recording sounds from the patient.

6. The apparatus according to one of claims 1 to 5, **characterized in that** on a support device (9) - preferably provided with a housing - on which the display device is movably mounted, an additional display device (10) is mounted - preferably rigidly or pivotably.

7. The apparatus according to one of claims 1 to 6, **characterized in that** the support (2) for the patient (1) is preferably designed as a multi-part lounger, the positioning of the parts of which is adjustable - preferably motor-adjustable, wherein the lounger is preferably configured in three parts, with a chest part, a middle part and a foot part.

8. The apparatus according to one of claims 1 to 7, **characterized in that** the measuring devices comprise at least two of the following measuring devices:
- an electrocardiography (ECG) apparatus,
- a fine current measuring apparatus,
- an apparatus for measuring the oxygen saturation in the blood,
- an infrared thermoscan apparatus
- a blood pressure measuring apparatus,
- an apparatus for measuring heart rate variability (HRV),
- an apparatus for analyzing the blood,
- an apparatus for frequency analysis of organs,
- an apparatus for measuring photons from biological tissue,
- an apparatus for magnetic and impedance measurement of biological tissue,
- an apparatus for examining the skin and/or teeth by means of imaging,
- an apparatus for examination and analysis of breathing air,
- an ultrasonic measuring apparatus,
- an electroencephalography apparatus (EEG),
- an electromyography apparatus (EMG),
- an electroneurography apparatus (ENG),
- a patient-operable input apparatus,
- an apparatus for video recording the patient's gait.

9. The apparatus according to one of claims 1 to 8, **characterized in that** the electronic evaluation apparatus to which, on one hand, mental measurement data which reflect the psychological state of the patient, and, on the other hand, physical measurement data which reflect the physical state of the patient is fed, wherein the electronic evaluation apparatus (5) is designed to determine output signals from the mental measurement data and the physical measurement data and their interrelationship.

10. The apparatus according to claim 9, **characterized in that** the mental measurement data on one hand, and the physical measurement data on the other hand, partially originate from different measuring devices.

11. The apparatus according to claim 10, **characterized in that** the physical measurement data originate from at least one of the following measuring devices:
- an electrocardiography (ECG) apparatus,
- a fine current measuring apparatus,
- an apparatus for measuring oxygen saturation in the blood,
- an infrared thermoscan apparatus
- a blood pressure measuring apparatus,
- an apparatus for measuring heart rate variability (HRV),
- an apparatus for analyzing the blood,
- an apparatus for organ frequency analysis,
- an apparatus for measuring photons from biological tissue,
- an apparatus for magnetic and impedance measurement of biological tissue,
- an apparatus for examining the skin and/or teeth by means of imaging,
- an apparatus for examining and analyzing the respiratory air,
- an ultrasonic measuring apparatus,
- an electromyography (EMG) apparatus,
wherein the mental measurement data come from at least one of the following measuring devices
- an electrocardiography (ECG)apparatus,
- a fine current measuring apparatus,
- an apparatus for measuring heart rate variability (HRV),
- an apparatus for organ frequency analysis,
- an apparatus for measuring photons from biological tissue,
- an electroencephalography apparatus (EEG),
- an electromneurography apparatus (ENG),
- a patient-operable input apparatus,
- an apparatus for video recording the patient's gait.

12. The apparatus according to one of claims 1 to 11, **characterized in that** an apparatus for - preferably non-invasive and contactless - influence on the patient is provided, preferably comprising a screen (3) and/or a speaker, wherein at least one measuring device is designed to record the patient's reaction and output reactive measurement data based thereon.

13. The apparatus according to claim 12, **characterized in that** it has an input device operable by the patient, in particular in the form of a touch screen (3), wherein the reactive measurement data correspond to the input data entered in the input device.

14. The apparatus according to claim 12 or 13, **characterized in that** the apparatus is designed such that the reactive measurement data, and preferably only this, are used as mental measurement data.

15. The apparatus according to one of claims 1 to 14, **characterized in that** at least one therapy device (15) is provided, to which at least part of the output signals of the electronic evaluation apparatus (5) is supplied as control signals, as a function of which the therapy device operates differently.

16. The apparatus according to one of claims 1 to 15, **characterized in that** at least one display device (14) for the physician, at least one display device (10) for the patient and/or at least one printing device (28) are provided, which are fed with output signals from the respective electronic evaluation apparatus (5) for graphically processed display and/or printing.

## Revendications

1. Système servant à l'examen d'un patient (1) humain avec un support (2) pour le patient (1), au moins un dispositif d'affichage (3) - réalisé en particulier en tant qu'écran -, qui est visible par le patient (1) se trouvant sur le support (2), au moins deux dispositifs de mesure (4) destinés à détecter des données de mesure du patient (1), qui fournissent des données de mesure concernant l'état du patient, ainsi qu'un système d'évaluation (5) électronique, qui est en liaison avec le dispositif d'affichage (3) et le dispositif de mesure (4) et qui est conçu pour déterminer des signaux de sortie en fonction de données de mesure provenant d'au moins deux dispositifs de mesure différents et de leur corrélation, dans lequel au moins un dispositif d'affichage et au moins un capteur (4a) d'un dispositif de mesure (4) sont montés de manière mobile par rapport au support (2) pour le patient (1), **caractérisé en ce que** l'au moins un dispositif d'affichage (3) et l'au moins un capteur (4a) d'un dispositif de mesure (4) sont montés de manière à pouvoir être déplacés de manière motorisée sur un dispositif de support (9), et que l'au moins un dispositif de mesure (4) comprend un scanner thermique médical.

2. Système selon la revendication 1, **caractérisé en ce qu'**au moins un dispositif d'affichage et au moins un capteur (4a) d'un dispositif de mesure (4) sont montés de manière à pouvoir être déplacés sur un système de maintien commun (6), en particulier un chariot monté de manière à pouvoir être déplacé.

3. Système selon la revendication 2, **caractérisé en ce que** le système de maintien (6) présente une partie de base pouvant être déplacée et une partie pivotante montée de manière à pouvoir pivoter sur celle-ci, qui supporte l'au moins un dispositif d'affichage (3) et l'au moins un capteur (4a) d'un dispositif de mesure (4).

4. Système selon l'une quelconque des revendications 1 à 3, **caractérisé en ce qu'**un dispositif d'entrée pouvant être utilisé par le patient, en particulier sous la forme d'un écran tactile, est associé au dispositif d'affichage.

5. Système selon l'une quelconque des revendications 1 à 4, **caractérisé en ce que** l'au moins un dispositif de mesure (4) comprend une caméra - adaptée en particulier pour le suivi oculaire - et/ou un microphone destiné à enregistrer des bruits du patient.

6. Système selon l'une quelconque des revendications 1 à 5, **caractérisé en ce qu'**un autre dispositif d'affichage (10) est monté - de préférence de manière rigide ou de manière à pouvoir pivoter - sur un dispositif de support (9) - pourvu de préférence d'un boîtier - sur lequel le dispositif d'affichage est monté de manière mobile.

7. Système selon l'une quelconque des revendications 1 à 6, **caractérisé en ce que** le support (2) pour le patient (1) est réalisé en tant que chaise longue en plusieurs parties, dont les parties peuvent être ajustées dans leur position - de préférence de manière motorisée **-,** dans lequel la chaise longue est réalisée de préférence en trois parties avec une partie poitrine, une partie centrale et une partie pieds.

8. Système selon l'une quelconque des revendications 1 à 7, **caractérisé en ce que** les dispositifs de mesure comprennent au moins deux des dispositifs de mesure suivants :
- un système d'électrocardiographie (ECG),
- un système de mesure de flux réduit,
- un système de mesure de saturation d'oxygène dans le sang,
- un système de balayage thermique infrarouge,
- un système de mesure de pression artérielle,
- un système de mesure de variabilité du rythme cardiaque (VRC),
- un système d'analyse du sang,
- un système d'analyse de fréquence d'organes,
- un système de mesure de photons provenant d'un tissu biologique,
- un système de mesure par magnétisme et impédance d'un tissu biologique,
- un système d'examen de la peau et/ou des dents au moyen d'une détection par imagerie,
- un système d'examen et d'analyse de l'air inspiré,
- un dispositif de mesure par ultrasons,
- un système d'électroencéphalographie (EEG),
- un système d'électromyographie (EMG),
- un système d'électroneurographie (ENG),
- un dispositif d'entrée pouvant être utilisé par le patient,
- un système de détection vidéo de la démarche du patient.

9. Système selon l'une quelconque des revendications 1 à 8, **caractérisé en ce que** d'une part des données de mesure mentales, qui reproduisent l'état psychique du patient, et d'autre part des données de mesure physiques, qui reproduisent l'état physique du patient, sont amenées au système d'évaluation électronique, dans lequel le système d'évaluation (5) électronique est conçu pour déterminer des signaux de sortie à partir des données de mesure mentales et des données de mesure physiques et de leur corrélation.

10. Système selon la revendication 9, **caractérisé en ce que** les données de mesure mentales d'une part et les données de mesure physiques d'autre part proviennent au moins en partie de différents dispositifs de mesure.

11. Système selon la revendication 10, **caractérisé en ce que** les données de mesure physiques proviennent au moins d'un des dispositifs de mesure suivants :
- un système d'électrocardiographie (ECG),
- un système de mesure de flux réduit,
- un système de mesure de saturation d'oxygène dans le sang,
- un système de balayage thermique infrarouge,
- un système de mesure de pression artérielle,
- un système de mesure de variabilité du rythme cardiaque (VRC),
- un système d'analyse du sang,
- un système d'analyse de fréquence d'organes,
- un système de mesure de photons provenant d'un tissu biologique,
- un système de mesure par magnétisme et impédance d'un tissu biologique,
- un système d'examen de la peau et/ou des dents au moyen d'une détection par imagerie,
- un système d'examen et d'analyse de l'air inspiré,
- un dispositif de mesure par ultrasons,
- un système d'électromyographie (EMG),
dans lequel les données de mesure mentales proviennent d'au moins un des dispositifs de mesure suivants :
- un système d'électrocardiographie (ECG),
- un système de mesure de flux réduit,
- un système de mesure de variabilité du rythme cardiaque (VRC),
- un système d'analyse de fréquence d'organes,
- un système de mesure de photons provenant d'un tissu biologique,
- un système de mesure par magnétisme et impédance d'un tissu biologique,
- un système d'électroencéphalographie (EEG),
- un système d'électroneurographie (ENG),
- un dispositif d'entrée pouvant être utilisé par le patient,
- un système de détection vidéo de la démarche du patient.

12. Système selon l'une quelconque des revendications 1 à 11, **caractérisé en ce qu'**un système est prévu pour influencer le patient - de préférence de manière non invasive et sans contact -, qui comprend de préférence un écran (3) et/ou un haut-parleur, dans lequel au moins un dispositif de mesure est conçu pour détecter la réaction du patient et pour fournir des données de mesure en réponse en fonction de celle-ci.

13. Système selon la revendication 12, **caractérisé en ce qu'**il présente un dispositif d'entrée pouvant être utilisé par le patient, en particulier sous la forme d'un écran tactile (3), dans lequel les données de mesure en réponse correspondent aux données d'entrée entrées dans le dispositif d'entrée.

14. Système selon la revendication 12 ou 13, **caractérisé en ce que** le système est conçu de telle sorte que les données de mesure en réponse, et de préférence uniquement celles-ci, sont utilisées en tant que données de mesure mentales.

15. Système selon l'une quelconque des revendications 1 à 14, **caractérisé en ce qu'**au moins un dispositif de traitement (15) est prévu, auquel au moins une partie des signaux de sortie du système d'évaluation (5) électronique sont amenés en tant que signaux de commande, en fonction desquels le dispositif de traitement fonctionne différemment.

16. Système selon l'une quelconque des revendications 1 à 15, **caractérisé en ce qu'**au moins un dispositif d'affichage (14) est prévu pour le médecin, au moins un dispositif d'affichage (10) est prévu pour le patient et/ou au moins un dispositif d'impression (28) est prévu, auxquels des signaux de sortie provenant du système d'évaluation (5) électronique sont amenés pour l'affichage graphique et/ou pour l'impression.
